# EUROPEAN PATENT APPLICATION

(11) **EP 2 711 003 A2**
(43) Date of publication of application: **26.03.2014**
(21) Application number: 12785468.5
(22) Date of filing: 16.05.2012
(51) Int. Cl.: A61K 31/047, A61K 31/20, A61K 31/045, A61P 1/00

(54) **COMPOUND USEFUL FOR PREVENTING OR TREATING IRRITABLE BOWEL SYNDROME AND COMPOSITION INCLUDING SAME**

(30) Priority: 18.05.2011 KR 20110046999
(71) Applicant: SK Chemicals Co., Ltd., Seongnam-Si, Gyeonggi-Do 463-400 (KR)
(72) Inventor: SUN, Won Suk, Seoul 120-786 (KR); JEON, Hyo Jin, Seongnam-si Gyeonggi-do 463-891 (KR); MIN, Dongsun, Seongnam-si Gyeonggi-do 463-731 (KR); KIM, Woong Sik, Suwon-si Gyeonggi-do 443-800 (KR); KIM, Taek Su, Suwon-si Gyeonggi-do 440-840 (KR); KUM, Do-Seung, Yongin-si Gyeonggi-do 448-971 (KR); RYU, Keun-Ho, Seoul 151-770 (KR); RHEE, Hae-In, Yongin-si Gyeonggi-do 448-918 (KR); PARK, Yang Hae, Seoul 140-713 (KR); SON, Hyun-Joo, Seoul 137-904 (KR); PARK, Eun-Ju, Suwon-si Gyeonggi-do 440-851 (KR); LEE, Bong-Yong, Seoul 137-935 (KR); NAMGUNG, Hojin, Seoul 137-950 (KR); PARK, Minseok, Seongnam-si Gyeonggi-do 461-380 (KR); KANG, Minseok, Seoul 151-927 (KR)
(74) Representative: Peters, Hajo
(86) International application number: PCT/KR2012/003828
(87) International publication number: WO 2012/157949

(57) **Abstract**

The present invention provides a compound of formula (I) or a prodrug thereof useful for treating or preventing irritable bowel syndrome, and a composition comprising the compound as an active ingredient. Also, the present invention provides a method for treating or preventing irritable bowel syndrome, which comprises administrating a therapeutically or prophylactically effective amount of the compound or the composition to a subject in need of treating or preventing irritable bowel syndrome.

## Description

### CROSS-REFERENCE TO RELATED APPLICATION

This application claims priority to Korean Patent Application No. 10-2011-0046999 filed in the Republic of Korea on May 18, 2011, the entire contents of which are incorporated herein by reference.

### TECHNICAL FIELD

The present invention relates to a compound for preventing or treating irritable bowel syndrome, a pharmaceutical composition comprising the compound and the medical-use thereof, and a method for preventing or treating irritable bowel syndrome using the compound.

### BACKGROUND ART

Irritable bowel syndrome (IBS) is a chronic disease accompanied by abdominal pain, abdominal discomfort such as long term-repeated abdominal distention, and abnormal tendencies of diarrhea, constipation, etc., in the absence of any detectable organic cause. The symptoms of IBS may worsen by psychological factors or stress. IBS is classified as diarrhea-predominant IBS, constipation-predominant IBS and pain-predominant IBS and the treatment of IBS is performed based on the symptoms. 30.8 % of Korean IBS patients are diarrhea-predominant IBS, 24.6 % are constipation-predominant IBS, and 44.6% have alternating stool pattern of diarrhea and constipation.

Medicine for treating IBS can be divided into medicine for treating one symptom and medicine for relieving overall symptoms. Medicine for treating abdominal pain includes smooth muscle relaxant, antidepressant, opioid agonist, etc.; medicine for treating constipation-predominant IBS includes fiber preparation, alleviator, 5-HT4 agonist, etc.; and medicine for treating diarrhea-predominant IBS includes antidiarrheal, 5-HT3 antagonist, etc. However, since the use of known drugs related to 5-HT are very limited due to their side-effects, there are only a limited amount of drugs that treat IBS. As a result, the treatment and relief of IBS symptoms depend highly on public remedies, which are not satisfactory.

That is, IBS is clearly different from a simple abdominal pain, diarrhea, or constipation because IBS is a chronic disease accompanied by diverse symptoms, for example, abdominal pain, abdominal distention, etc. with defecation abnormalities such as constipation and diarrhea. Therefore, in order to evaluate the therapeutic effects of IBS, various symptoms including pain-relieving effect, improvement of defecation abnormality, etc should be evaluated together. Such an evaluation of the therapeutic effect for IBS has been conducted by using a CRD (colorectal distension) model, a representative evaluation model of IBS.

Meanwhile, neurokinin (NK) receptor is classified as subtypes of NK1, NK2 and NK3, and is a receptor binding with tachykinin family, which is neuropeptides acting on the central nervous system and the peripheral nervous system, for example, substance P (SP), Neurokinin A and Neurokinin B. This NK receptor is present in the central nervous system such as the brain's amygdala, hippocampus, hypothalamus, corpus striatum and spinal cord, or the peripheral nervous system such as the skin, inflammatory system, digestive system, respiratory system, cardiovascular system, etc., and is closely associated with bowel movements and visceral hypersensitivity *(see* JH. La et al., World J. Gastroenterol., 11(2), p 237-241, 2005; MS Kramer, Science, 281(5383) p 1624-1625, 1998; and G. J. Sanger., Br. J. Pharmacol., 141, p 1303-1312, 2004). Recently, based on the physiological function of the NK receptor, antagonists of the NK receptor have been studied to develop new drugs for IBS *(see* R. A. Duffy, Expert Opin. Emerg. Drugs, 9(1), 2004; M. Camilleri, Br. J. Pharmacol., 141, p 1237-1248, 2004; G. J. Sanger., Br. J. Pharmacol., 141, p 1303-1312, 2004; and A. Lecci et al., Br. J. Pharmacol., 141, p 1249-1263,2004).

### DISCLOSURE

### Technical Problem

Accordingly, the object of the present invention is to provide a compound useful for treating or preventing irritable bowel syndrome, a pharmaceutical composition comprising the compound and a medical use thereof, and a method for treating or preventing irritable bowel syndrome by using the compound.

### Technical Solution

To achieve the object, the present invention provides a composition (preferably a pharmaceutical composition) for treating or preventing irritable bowel syndrome (IBS), comprising a compound of the following formula (I) as an active ingredient: 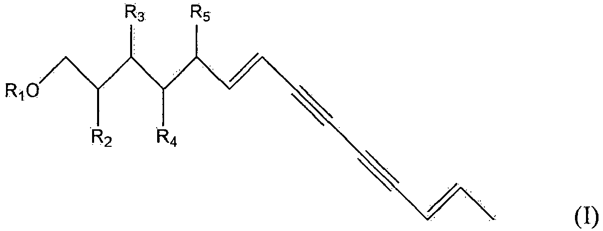 wherein,
R₁ is hydrogen, acetyl, C₁-C₆ alkyl, substituted C₁-C₆ alkyl, phenyl or substituted phenyl, and
R₂ to R₅ are each independently hydrogen or hydroxyl,
the substituted alkyl or substituted phenyl is substituted with at least one substituent selected from the group consisting of hydroxyl and C₁-C₃ alkyl at one or more positions.

Preferably, R₁ is hydrogen, acetyl or C₁-C₆ alkyl, and R₂ to R₅ are each independently hydrogen or hydroxyl.

More preferably, R₁ is hydrogen or acetyl, R₃ is hydroxyl, and R₂, R₄ and R₅ are hydrogen.

Most preferably, the present invention provides a composition, preferably a pharmaceutical composition for treating or preventing IBS, comprising (6E,12E)-3-hydroxytetradeca-6,12-dien-8,10-diyn-1-yl acetate which is the compound of formula (I) wherein R₁ is acetyl, R₃ is hydroxyl, and R₂, R₄ and R₅ are hydrogen, as an active ingredient.

The present inventors have endeavored to develop a substance useful for treating or preventing IBS and found that the compounds of formula (I) can inhibit NK2 receptors, motilin receptors, melanocortin MC1 receptors, cannabinoid CB₂ receptors and so on which are associated with bowel movements and visceral hypersensitivity, as well as inhibit the activity of guanylate cyclase which is associated with bowel movements and visceral hypersensitivity, and exhibit a superior effect in a CRD model which is a representative evaluation model of IBS.

Among various receptors including serotonin and NK which have been studied to treat IBS patients, the motilin receptors associated with bowel movements are reported to improve the abnormal movement of gastrointestinal tract, thereby suppressing IBS *(see* L. Ohman et al., Digestive and Liver Disease, Volume 39, Issue 3, p 201-215, 2007). Also, the cannabinoid receptors are reported to suppress and decrease pain inhibition in visceral hypersensitivity which are main measurement indexes of a CRD model (*see* Gerard Clarke et al, Trends in Molecular Medicine, Volume 15, Issue 10, p 478-489, 2009). Meanwhile, the melanocortin MC1 receptors are reported to have antiinflammatory effects in colitis and pain control and thus expected to be multiple symptoms of IBS *(see* C. Maaser et al, Gut. 55(10) p 1415-1422. Epub., 2006). Granulate cyclase is one of the targets associated with bowel movements and linaclotide, which is known as an agonist of the guanylate cyclase, developed as a therapeutic agent for IBS, and so a linaclotide functioning as an antagonist or agonist of the guanylate cyclase is expected to improve defecation abnormalities (see Jeffrey M. Johnston et al., Gastroenterology, Volume 139, Issue 6, p 1877-1886.e2, 2010).

The compound of formula (I) which is comprised as an active ingredient in the composition for treating or preventing IBS according to the present invention may be obtained by a chemical synthetic method well-known in the art, or by an extraction and/or purification, well-known in the art, of *Atractylodes japonica* (preferably, rhizome).

For example, (6E,12E)-3-hydroxytetradeca-6,12-dien-8,10-diyn-1-yl acetate, a preferred example of the compound of formula (I) according to the present invention, may be obtained by extracting *Atractylodes japonica* with C₁-C₃ lower alcohol (S1), fractionating the extract with water and EtOAc (S2), fractionating again the EtOAc fraction with *n*-hexane and 30 % (v/v) of aqueous methanol or ethanol solution (S3), and separating and purifying the n-hexane fraction by column (silica and C₁₈ columns) liquid chromatography, and the present invention is not limited to such a preparation method.

In addition, the present invention includes a solvate, particularly a hydrate form, and an unsolvated form of the compound of formula (I). The compound of the present invention may exist in a crystalline or amorphous form, and all physical forms thereof are incorporated within the scope of the present invention. Also, the compound of the present invention may contain asymmetric carbon atoms (chiral center) and double bonds depending on the form of the compounds and may include racemic mixtures, enantiomer, diastereomer and geometrical isomer thereof, which are incorporated within the scope of the present invention.

Also, a prodrug of the compound of formula (I), which may be produced by a reaction such as hydrolysis *in vivo,* is incorporated within the equivalent scope of the present invention. For example, the prodrug may include, but is not limited to, compounds wherein
- R₁ is substituted with -CO-H, 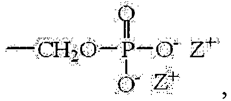 or -O-(C=O)-R" (wherein, R" is C₁-C₆ alkyl, substituted C₁-C₆ alkyl, phenyl, substituted phenyl, or 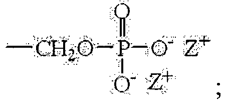 Z is hydrogen, alkali metal or amine; and the substituted alkyl or substituted phenyl is substituted with at least one substituent selected from the group consisting of hydroxyl and C₁-C₃ alkyl at one or more positions), or
- H existed in hydroxyl of R₂ to R₅ is substituted with -CO-H, R' or -O-(C=O)-R" (wherein, R' and R" are each independently C₁-C₆ alkyl, substituted C₁-C₆ alkyl, phenyl, substituted phenyl, or 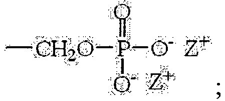 Z is hydrogen, alkali metal or amine; and the substituted alkyl or substituted phenyl is substituted with at least one substituent selected from the group consisting of hydroxyl and C₁-C₃ alkyl at one or more positions), or
- two among -OR₁ and hydroxyl of R₂ to R₅ may be taken together to form a ring connected in the -O-X-O- structure (wherein, X is C₁-C₆ alkyl, substituted C₁-C₆ alkyl, C=O, NH, -(CH₂)nO-C=O- or -C=O-O(CH₂)n-; n is an integer of 1 to 6; and the substituted alkyl is substituted with at least one substituent selected from the group consisting of hydroxyl and C₁-C₃ alkyl at one or more positions).

In addition, the compound of formula (I) of the present invention or a prodrug thereof may be administered in the form of a pharmaceutically acceptable salt. The term "pharmaceutically acceptable salt" as used herein means a non-toxic addition salt prepared by using an acid or base. When the compound of the present invention is relatively acidic, a base-addition salt thereof may be obtained by contacting the neutral form of the compound with a sufficient amount of the desired base in a suitable inert solvent. The pharmaceutically acceptable base-addition salt may include, but is not limited thereto, salts with an inorganic base such as lithium, sodium, potassium, calcium, ammonium and magnesium or an organic base such as amine. When the compound of the present invention is relatively basic, an acid-addition salt thereof may be obtained by contacting the neutral form of the compound with a sufficient amount of the desired acid in a suitable inert solvent. The pharmaceutically acceptable acid-addition salt may include, but is not limited thereto, salts with propionic acid, isobutylic acid, oxalic acid, malic acid, malonic acid, benzoic acid, succinic acid, suberic acid, fumaric acid, mandelic acid, phthalic acid, benzensulfonic acid, *p*-tolylsulfonic acid, citric acid, tartaric acid, methansulfonic acid, hydrochloric acid, bromic acid, nitric acid, carbonic acid, monohydrogencarbonic acid, phosphoric acid, monohydrogenphosphoric acid, dihydrogenphosphoric acid, sulfuric acid, monohyrogen sulfuric acid, hydrogen iodide or phosphorous acid. Also, the acid-addition salt may include, but is not limited to, amino acid salts such as arginate and salts of organic acid analogues such as glucuronic acid or galactunoric acid.

Furthermore, the present invention provides a pharmaceutical composition or health food composition, preferably a pharmaceutical composition for treating or preventing irritable bowel syndrome, which comprises the compound of formula (I), a prodrug, isomer, solvate, hydrate or pharmaceutically acceptable salt thereof together with pharmaceutically acceptable excipients or additives. The compounds of the present invention may be administered alone or by mixing with a suitable carrier or excipient and it may be administered in single dose or divided doses.

The composition of the present invention may be formulated in a solid or liquid form. The solid formulation form may include, but is not limited to, powders, granules, tablets, capsules and suppositorys. The solid formulation may comprise, but is not limited to, excipients, flavoring agents, binders, preservatives, disintegrants, glidants and fillers. The liquid formation form may include, but is not limited to, water, solutions such as propylene glycol solution, suspensions and emulsions, which may be prepared by mixing with suitable coloring agents, flavoring agents, stabilizers and viscosity-increasing agent.

For example, a powder formulation may be prepared by simply mixing the compound of the present invention with suitable pharmaceutically acceptable excipients such as sucrose, starch and microcrystalline cellulose. A granule formulation may be prepared by mixing the compound of the present invention; suitable pharmaceutically acceptable excipients; suitable pharmaceutically acceptable binders such as polyvinyl pyrrolidone and hydroxypropyl cellulose, followed by wet granulation method using a solvent such as water, ethanol and isopropanol, or dry granulation method using compression force. Also, a tablet formulation may be prepared by mixing the granule formulation with suitable pharmaceutically acceptable glidants such as magnesium stearate, followed by tabletting using a tablet machine.

The pharmaceutical composition of the present invention may be administered in various forms of an oral, injection (e.g., intramuscular, intraperitoneal, intraveneous or subcutaneous injections, infusion and implant), inhalation, nasal, vaginal, rectal, sublingual, transdermal or topical administration depending on diseases to be treated and the severity of the diseases, but it is not limited thereto. The composition of the present invention may be formulated in a suitable unit dose formulation comprising non-toxic pharmaceutically acceptable carriers, additives and vehicles. A depot formulation which can continuously release a drug for a certain period is also incorporated in the scope of the present invention.

The composition of the present invention contains 0.00001 to 50 wt%, preferably 0.001 to 40 wt%, more preferably 0.01 to 30 wt%, and most preferably 0.1 to 20 wt% of the compound of formula (I) or a prodrug thereof based on the total weight of the composition.

Furthermore, the present invention provides a method for treating or preventing irritable bowel syndrome, which comprises administrating to a subject in need of treating or preventing irritable bowel syndrome a therapeutically or prophylactically effective amount of the compound of formula (I), a prodrug, isomer, solvate, hydrate or pharmaceutically acceptable salt thereof.

The suitable daily dose for the treatment or prevention of irritable bowel syndrome is about 1 mg/kg to about 1,200 mg/kg, preferably 50 mg/kg to about 600 mg/kg of the compound of formula (I) according to the present invention, a prodrug, isomer, solvate, hydrate or pharmaceutically acceptable salt thereof. However, the dose may vary depending on the conditions of the patients (age, sex, weight, etc.), the severity of the state which has been treated and compounds used, and if it is necessary, can be administered in divided doses for a day.

The compound of formula (I) according to the present invention, a prodrug, isomer, solvate, hydrate or pharmaceutically acceptable salt thereof, or the composition comprising the same as an active ingredient can treat or prevent at least one irritable bowel syndrome (IBS) selected from diarrhea-predominant IBS, constipation-predominant IBS and pain-predominant IBS.

Also, the present invention provides a method for inhibiting NK (neurokinin) 2 receptors, motilin receptors, melanocortin MC1 receptors or cannabinoid CB₂ receptors, which comprises administrating the compound of formula (I), a prodrug, isomer, solvate, hydrate or pharmaceutically acceptable salt thereof to a mammal including a human-being.

In addition, the present invention provides a method for inhibiting the activity of guanylate cyclase, which comprises administrating the compound of formula (I), a prodrug, isomer, solvate, hydrate or pharmaceutically acceptable salt thereof to a mammal including a human being.

### Advantageous Effects

The present invention provides an active ingredient useful for treating or preventing irritable bowel syndrome and a composition comprising the active ingredient. The present invention also provides a method for treating or preventing irritable bowel syndrome, which comprises administrating the compound of the present invention or a composition comprising the compound in a therapeutically or prophylactically effective amount to a subject in need of treating or preventing irritable bowel syndrome.

### DESCRIPTION OF DRAWINGS

FIG. 1 is an ultra performance liquid chromatography (UPLC) result of (6E,12E)-3-hydroxytetradeca-6,12-dien-8,10-diyn-1-yl acetate, a preferred example of the compounds according to the present invention after column purification.
FIG. 2 is a graph showing the effect of (6E,12E)-3-hydroxytetradeca-6,12-dien-8,10-diyn-1-yl acetate, a preferred example of the compounds of the present invention, in the CRD model.

### BEST MODE

Hereinafter, the present invention will be described in further detail with reference to Examples. However, the following examples are offered by way of illustration to help those skilled in the art understand the present invention, and are not intended to limit the scope of the invention. It is apparent that various changes may be made without departing from the spirit and scope of the invention.

### <Separation and Purification of the Compound of the Present Invention>

(6E,12E)-3-hydroxytetradeca-6,12-dien-8,10-diyn-1-yl acetate, an example of the compounds according to the present invention, was obtained as follows.

### Extraction and Solvent Fraction

1 kg of *Atractylodes japonica* rhizome was extracted in 5 L of methanol at room temperature for 72 hours, filtered with a filter paper (Whatman 1, 11 *µ*m), and concentrated under reduced pressure at 50 °C to obtain a methanol extract (yield: about 11.8 % relative to the crude drug).

The concentrated methanol extract was suspended in 5 L of distilled water and fractionated twice with 5 L of ethyl acetate to remove the distilled water fraction (yield: about 6.7 % relative to the crude drug) and concentrated under reduced pressure at 50 °C to obtain an ethyl acetate fraction (yield: about 5.1 % relative to the crude drug).

The concentrated ethyl acetate fraction was suspended in 5 L of 30 % (v/v) aqueous methanol solution and fractionated twice with 5 L of *n*-hexane fraction to remove the 30 % (v/v) aqueous methanol solution fraction (yield: about 0.7 % relative to the crude drug) and concentrated under reduced pressure at 50 °C to obtain a *n*-hexane fraction (yield: about 4.4 % relative to the crude drug).

### Separation and Purification using Column

The *n*-hexane fraction prepared above was dissolved in *n*-hexane to obtain a 50 mg/mℓ solution and fractionated with normal phase MPLC (Biotage , Isolera one) using silica cartridge (Biotage, HP-Sil SNAP Flash Cartridge 100g, Lot No. 10011910C) (Conditions: 50 mℓ/min of flow rate; *n*-hexane/ethyl= 100%/0% ∼ 70%/30% ∼ 20%/80% ∼ 10%/90% ∼ 0%/100%; 240 mℓ of max fraction volume; 500 Mℓ of initial waste; UV 220 nm). The sixth fraction was concentrated under reduced pressure at 50 °C to obtain the desired fraction.

The fraction concentrated was dissolved in methanol to obtain a 50 mg/mℓ solution and further purified with reversed phase MPLC (Biotage , Isolera one) using C₁₈ cartridge (Biotage, KP-C18-HS SNAP Flash Cartridge 120g) (Conditions: 50 mℓ/min of flow rate; distilled water/methanol = 50%150% ∼ 30%/70% ∼ 0%/100%; 240 mℓ of max fraction volume; 500 mℓ of initial waste; UV 220 nm). The first to third fractions were recovered and concentrated under reduced pressure at 50 °C to obtain the desired fraction.

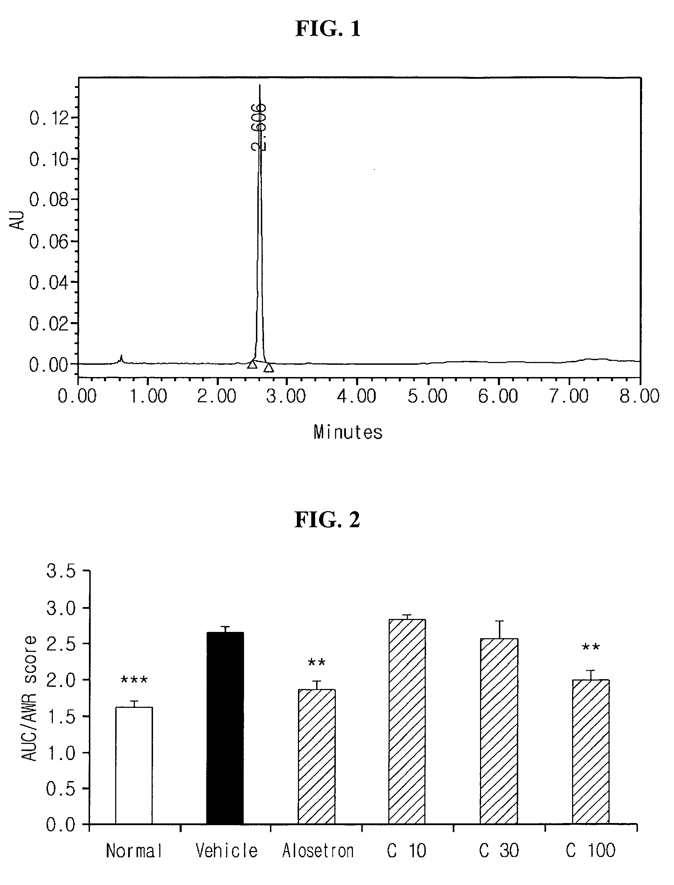

The fraction obtained was dissolved in methanol to obtain a 50 mg/mℓ solution and purified twice with preparative HPLC (Hitachi) using C₁₈ column (YMC-Pack Pro C18 RS, 250x20mm I.D., S - 5 *µ*m, 8 nm, No. 2025000314) (9.5 mℓ/min of flow rate; distilled water : acetonitrile = 30 : 70; UV 220 nm) to obtain about 200 mg of the desired compound (yield: about 0.02 % relative to the crude drug). FIG. 1 is a UPLC result of the compound thus obtained.

### <Structural Analysis of the Compound of Present Invention>

The finally separated compound of the present invention was analyzed for its structure with UV, NMR (Bruker, Avance 600; ¹H-NMR, ¹³C-NMR, HMQC, HMBC, COSY, DEPT, TOCSY, NOESY) and GC/MS (PerkinElmer Clarus 600 series: Column Elite-5MS, 30 m, 0.25 mm ID, 0.25*µ*m df).

### UV and NMR Analysis

The compound of the present invention exhibited specific polyacetyle-based absorbance patterns having λmax (nm) of 312, 293, 276 and 231 in UV spectrum.

The results of NMR analysis using ¹H-NMR and ¹³C-NMR are shown below.

¹H-NMR (600 MHz, CDCl₃) : δH 1.56 (2H, dt, J=8.5, 6.7, 3.1 Hz, H-4), 1.67 (1H, ddt, J=14.4, 9.7, 5.0 Hz, H-2), 1.79 (1H, m, H-2), 1.82 (3H, dd, J=6.9, 1.6 Hz, H-14), 2.07 (3H, s, acetyl Me), 2.24 (1H, dt, J=14.9, 7.3 Hz, H-5), 2.32 (1H, dt, J=14.8, 7.4 Hz, H-5), 3.66 (1H,d, J=3.2 Hz, H-3), 4.12 (1H, dt, J=11.1, 5.4 Hz, H-1), 4.37 (1H, ddd, J=11.3, 8.9, 4.7 Hz, H-1), 5.56 (1H, d, J=13.6 Hz, H-7), 5.59 (1H, d, J=14.0 Hz, H-12), 6.3 (2H, m, H-6, H-13).

¹³C-NMR (600 MHz, CDCl₃) : δC 18.93 (C-14), 21.01 (acetyl Me), 29.48 (C-5), 36.03 (C-4), 36.47 (C-2), 61.57 (C-1), 67.77 (C-3), 72.94 (C-9), 72.30 (C-10), 79.47 (C-8), 79.96 (C-11), 109.30 (C-7), 109.90 (C-12), 143.48 (C-13), 147.41 (C-6), 171.6 (acetyl C=O).

From the ¹³C-NMR spectrum, it is confirmed that the compound of the present invention has 16 carbon atoms in total, which are 5 quaternary carbons (δC 171.6, 79.96, 79.47, 73.94, 72.3), 5 methyne carbons (δC 147.41, 143.48, 109.9, 109.3, 67.77), 4 methylene carbons (δC 61.57, 36.47, 36.03, 29.48), and 2 methyl carbons (δC 21.01, 18.93) as confirmed from DEPT.

Among 5 quaternary carbons, δC 171.6 is a carbonyl carbon, and the remaining 4 carbons are expected to form acetylene bonds. From the ¹H-NMR and HMQC spectrums, 19 proton peaks were paired with each carbon peak. Among 5 methyne carbons, it is confirmed that carbon at δ67.77 was paired with proton at δ3.66, which exhibits the bonding with one hydroxyl, and the remaining carbons are expected to be olefinic carbons.

From COSY, HMQC, and HMBC spectrums, the presence of acetoxy group and the relative position thereof were confirmed. In the acetoxy group, it was confirmed by HMQC correlation that both the proton (δH 2.07; H-2') of methyl was adjacent to the carbonyl carbon (δC 171.6; C-1') and the H-1 proton (δH 4.37, 4.12) was adjacent to the carbonyl carbon (δC 171.6; C-1'). Also, it is confirmed that the shift values (δC 61.57, δH 4.37, 4.12) of C-1 carbon and H-1 proton were relatively in down field by the acetoxy group.

In the COSY spectrum, H-4 proton (δH 1.56) was coupled with H-3 proton (δH 3.66) and H-5 proton (δH 2.32, 2.24), and the H-5 proton (δH 2.32, 2.24) was also confirmed to be correlated with C-4 carbon (δC 36.03) in the HMBC spectrum. As can be seen from the COSY spectrum, the H-4 proton (δH 1.56) was coupled with H-6 proton (δH 6.3) and H-7 proton (δH 5.56), which are adjacent to the H-5 proton (δH 2.32, 2.24).

### GC/MS Analysis

Molecular ion peaks (m/z 260) were confirmed by GC/MS, and unsaturated hydrocarbon chain fragments including double or triple bonds were confirmed at base peak (m/z 128). The fragmentation pattern results are shown in Table 1.

**Table 1**

| m/z | Fragment |
|---|---|
| 260 | M⁺ |
| 199 | M⁺-CH₃COOH-H |
| 165 | M⁺-[C₁]-H₂O-4H |
| 153 | M⁺-[C₁+C₂]-H₂O-2H |
| 141 | M⁺-[C₁+C₂+C₃]-2H |
| 128 | M⁺-[C₁+C₂+C₃+C₄]-3H |
| 115 | M⁺-[C₁+C₂+C₃+C₄+C₅] |

From analysis results above, the compound which was separated and purified above was confirmed as being (6E,12E)-3-hydroxytetradeca-6,12-dien-8,10-diyn-1-yl acetate, a preferred example of the compounds according to the present invention.

### <In vitro Mechanism Study>

*In vitro* study on the mechanism of the separated (6E,12E)-3-hydroxytetradeca-6,12-dien-8,10-diyn-1-yl acetate was performed as follows:

### Evaluation of Inhibiting Activity against NK2 Preceptor

Each of 100 *µ*ℓ of a modified HEPES buffer was added to each well, to which the compound (50, 500, 1500, 5000, 15000 µM) dissolved in DMSO and a positive control, MEN-10,376 (0.5, 2.5, 5, 25, 50 µM) were added in an amount of 5 *µ*ℓ*,* respectively. Thereto, each of 50 *µ*ℓ of a NK2 receptor ligand, [³H]SR-48968 was added, and each of 100 *µ*ℓ of a modified HEPES buffer having cell membranes in which a human-derived NK2 receptor is expressed was added. After incubating with stirring (200 rpm) at 25 °C for 90 minutes, the resultants were filtered and washed 3 times.

The number of cell membranes bound with an isotope ([³H]SR-48968) was calculated by means of β-ray measurement. At this time, as a test material is strongly bound with the receptor, the ligand [³H]SR-48968 is not bound and measured values decrease (competitive bonding). The value for the well added with only the ligand was represented as "0% inhibition", and the well having no ligand, as "100% inhibition', and the inhibition rate was measured depending on the amount of drug concentration.

### Evaluation of Inhibiting Activity against Motilin Receptor

Each of 100 *µ*ℓ of a modified Tris-HCl buffer was added to each well, to which the compound (50, 500, 1500, 5000, 15000 µM) of the present invention dissolved in DMSO and a positive control, motilin (2.5, 5, 25, 50, 250 nM) were added in an amount of 5 *µ*ℓ, respectively. Thereto, each of 50 *µ*ℓ of a motilin receptor ligand, [³H]SR-48968 was added, and each of 100 *µ*ℓ of a modified Tris-HCl buffer having cell membranes in which a human-derived motilin receptor is expressed was added. After incubating at 25 °C for 150 minutes, the resultants were filtered and washed 3 times. The number of cell membranes bound with an isotope was calculated by means of P-ray measurement.

### Evaluation of Inhibiting Activity against Melanocortin MC1 Receptor

Each of 100 *µ*ℓ of a modified HEPES-KOH buffer was added to each well, to which the compound (50, 500, 1500, 5000, 15000 µM) of the present invention dissolved in DMSO and a positive control, NDP-a-MSH (0.25, 0.5, 2.5, 5, 25 nM) were added in an amount of 5 *µ*ℓ*,* respectively. Thereto, each of 50 *µ*ℓ of a melanocortin MC1 receptor ligand, [¹²⁵I] NDP-a-MSH was added, and each of 100 *µ*ℓ of a modified Tris-HCl buffer having cell membranes in which a human-derived melanocortin MC1 receptor is expressed was added. After incubating at 37 °C for 120 minutes, the resultants were filtered and washed 3 times. The number of cell membranes bound with an isotope was calculated by means of β-ray measurement.

### Evaluation of Inhibiting Activity against cannabinoid CB₂ Receptor

Each of 100 *µ*ℓ of a modified HEPES buffer was added to each well, to which the compound (50, 500, 1500, 5000, 15000 µM) of the present invention dissolved in DMSO and a positive control, R(+)-WIN-55, 212-2 (0.05, 0.25, 0.5, 2.5, 5 nM) were added in an amount of 5 *µ*ℓ, respectively. Thereto, each of 50 *µ*ℓ of a cannabinoid CB₂ receptor ligand, [³H]WIN-55, 212-2 was added, and each of 100 *µ*ℓ of a modified Tris-HCl buffer having cell membranes in which a human-derived cannabinoid CB₂ receptor is expressed was added. After incubating at 37 °C for 90 minutes, the resultants were filtered and washed 3 times. The number of cell membranes bound with an isotope was calculated by means of β-ray measurement.

### Evaluation of Activity against or for Guanylate Cyclase

Plasmid in which a human-derived guanylate cyclase is expressed was transfected to Sf9 insect cells, followed by proliferation and then lysis. The compound (1, 10, 30, 100, 300 µM) of the present invention dissolved in DMSO and a positive control, ODQ (1H-[1,2,4]oxadiazolo[4,3-a]quinoxalin-1-one, 0.5, 0.1, 0.05, 0.01, 0.005 µM) were added to each well, to which guanylate cyclase (the lysed solution) which is mixed in a modified Tris-HCl buffer was added until its final concentration became to 0.013 *µ*g/mℓ. After incubating at 37 °C for 20 minutes, 1N HCl was added to end the reaction.

After removing the guanylate cyclase, an amount of cGMP produced from the reaction was subject to quantitative measurement using an EIA kit. The amount of cGMP increases as the guanylate cyclase converting GTP to cGMP is activated. The activation of the guanylate cyclase was calculated based on the assumption that the result of sodium nitroprusside (30µM), an agonist of the guanylate cyclase was 100%. The antagonistic activity against the guanylate cyclase was calculated based on the assumption that the result when treated only with 30µM of sodium nitroprusside was 100% compared to when treated with both sodium nitroprusside and a drug.

The results obtained above are shown in Table 2. From Table 2, it can be confirmed that (6E,12E)-3-hydroxytetradeca-6,12-dien-8,10-diyn-1-yl acetate, a preferred compound of the present invention has an inhibiting activity against cannabinoid CB₂ receptors, melanocortin MC1 receptors, NK2 receptors which are associated with the sensory function of bowel, and motilin receptors, guanylate cyclases, NK2 which are associated with the movement function of bowel.

**Table 2**

| Target | IC₅₀(µM) |
|---|---|
| NK2 receptor | 30.6 |
| motilin receptor | 29.1 |
| melanocortin MC1 receptor | 133 |
| cannabinoid CB₂ receptor | 45 |
| guanylate cyclase | 18.9 |

### <Evaluation on Suppressing Effect of Visceral Pain in CRD model>

To evaluate the suppressing effect of the separated (6E,12E)-3-hydroxytetradeca-6,12-dien-8,10-diyn-1-yl acetate on the visceral hypersensitivity, a Colorectal Distension (CRD) test that has been often used in the evaluation of drugs for IBS was conducted as follows (*see* JH. La et al., World J. Gastroenterol., Dec., 9(12): p 2791-2795, 2003).

250 to 300 g of Sprague-Dawley male rats (Charles River) were used for the CRD test. Two rats per one cage were reared in a room of 25°C, humidity 50%, and day-night 12:12 hours of cycle. Rats were freely provided with drink water and feed, and were adapted to the room condition for 5 days, followed by inducing colitis. Feed provision was stopped 24 hours prior to the induction of colitis, and the rats were subject to breathing anesthesia with ether and then inserted with Rubber catheter (PE 50) from the anus up to 8cm through rectum.

1 Mℓ of 3.5 % acetic acid (acetic acid in 0.9% saline) was administered though the catheter into the lumen of the colon, and then the anus was tied to block the leakage of the solution. After 30 seconds, 1 Mℓ of 0.9 % saline was administered through the same catheter into the lumen of the colon to wash away the acetic acid solution.

A 2 cm-long rubber balloon was inserted into the rectum of each rat, and was filled with 37°C-warmed water in stages, from 0.1 to 1.0 Mℓ. The appearing pain reactions of rats were recorded. The specific behaviors of CRD test animals were represented with AWR (abdominal withdrawal reflex), which was indirectly and quantitatively analyzed by AWR scores given on each behavior, and the AWR scores were used for identifying the abdominal pain reaction. The AWR scores obtained are shown in Table 3 *(see* E. D. Al-Chaer et al., Gastroenterology, Nov., 119(5), p 1276-1285. 2000).

**Table 3**

| AWR Score | Specific Behavior |
|---|---|
| 0 | No behavioral response to distension |
| 1 | Brief head movements followed by immobility during distension |
| 2 | Contraction of abdominal muscle without lifting of abdomen |
| 3 | Lifting of abdomen |
| 4 | Body arching and lifting of pelvic structure |

The presence of visceral hypersensitivity was checked in CRD rats 7 days after inducing colitis. Through this checking, model animals having symptoms like IBS were selected *(see* JH. La et al., World J. Gastroenterol., Dec., 9(12): p 2791-2795, 2003). Each (6E,12E)-3-hydroxytetradeca-6,12-dien-8,10-diyn-1-yl acetate according to the present invention was dissolved in 0.5% carboxymethylcellulose (CMC) water solution such that the concentrations of 10, 30 and 100 mg/kg were obtained, respectively, and each of them was orally administered to the rats selected. 20 mg/kg of positive control, alosetron HCL (Jiangyin Yongda Chemical Co., Ltd.) was orally administered. Then, rats were stabilized for about 1 hour, and then CRD tests were performed to record AWR score. AWR score according to distension volume (Mℓ) and its AUC (area under the curve) were calculated to quantify the reaction results of vehicle-administered group, positive control and the compound-administered group. Student's t-test (p<0.01 (**) or p<0.001 (***)) was used for statistical approach, and significance relative to the vehicle-administered group was detected. The results are shown in FIG. 2 (mean ± S.E., n ≥ 3).

In FIG. 2, "Normal" refers to a normal group without induced colitis, "Vehicle" refers to a colitis-induced group which is orally administered with only vehicle, and "C 10", "C 30" and "C 100" refer to colitis-induced groups which are orally administered with 10, 30 and 100 mg/kg of the compound of the present invention, respectively.

As shown in FIG. 2, the preferred compound of the present invention has a suppressing effect against visceral pain occurred in visceral hypersensitivity as its concentration increased, particularly, the group administered with 100 mg/kg of the compound according to the present invention represents a significant suppressing effect against visceral hypersensitivity relative to the vehicle group.

## Claims

1. A pharmaceutical composition for treating or preventing irritable bowel syndrome, comprising a compound of the following formula (I) as an active ingredient: 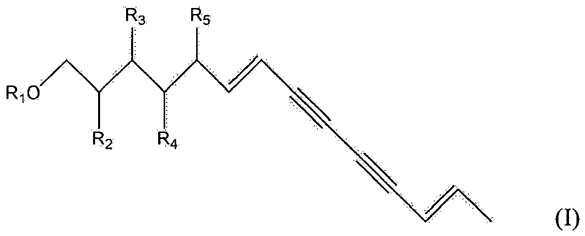 wherein,
R₁ is hydrogen, acetyl, C₁-C₆ alkyl, substituted C₁-C₆ alkyl, phenyl or substituted phenyl, and
R₂ to R₅ are each independently hydrogen or hydroxyl,
the substituted alkyl or substituted phenyl is substituted with at least one substituent selected from the group consisting of hydroxyl and C₁-C₃ alkyl at one or more positions.

2. The pharmaceutical composition for treating or preventing irritable bowel syndrome according to claim 1, wherein R₁ is hydrogen or acetyl, R₃ is hydroxyl, and R₂, R₄ and R₅ are hydrogen.

3. The pharmaceutical composition for treating or preventing irritable bowel syndrome according to claim 2, wherein R₁ is acetyl, R₃ is hydroxyl, and R₂, R₄ and R₅ are hydrogen.
